# FASCICULE DE BREVET EUROPEEN

(11) **EP 1 251 808 B1**
(45) Date de publication et mention de la délivrance du brevet: **10.12.2008**
(21) Numéro de dépôt: 01907649.6
(22) Date de dépôt: 19.01.2001
(51) Int. Cl.: A61F 5/00

(54) **ANNEAU DE GASTROPLASTIE A COMMANDE UNIQUE**
EINHAND-MANIPULIERBARER RING ZUR MAGENVERENGUNG
SINGLE CONTROL GASTRIC BAND

(30) Priorité: 20.01.2000 FR 0000693
(43) Date de publication de la demande: 30.10.2002
(73) Titulaire: Compagnie Européenne d'Etude et de Recherche de Dispositifs pour l'Implantation par Laparoscopie, 38200 Vienne (FR)
(72) Inventeur: BENCHETRIT, Salomon, F-69300 Caluire (FR)
(74) Mandataire: Martin, Didier Roland Valéry
(86) Numéro de dépôt international: PCT/FR2001/000185
(87) Numéro de publication internationale: WO 2001/052777

(56) Documents cités:
- EP-A- 0 611 561
- WO-A-94/27504
- DE-A- 19 751 733
- DE-U- 9 014 048
- US-A- 5 074 868

## Description

### DOMAINE DE L'INVENTION

La présente invention se rapporte au domaine technique des implants chirurgicaux destinés à traiter l'obésité par implantation d'une bande gastrique souple destinée à resserrer l'estomac d'un patient, ladite bande gastrique étant pourvue d'une chambre de compression annulaire à volume variable et réglable par l'intermédiaire d'un cathéter de réglage relié à un dispositif de réglage et de commande implanté dans le corps du patient.

La présente invention concerne un anneau de gastroplastie formé par une bande souple destinée à être fermée autour de l'estomac vers ses deux parties d'extrémité, à l'aide d'un système de fermeture pour réduire le diamètre de l'ouverture de stoma, ladite bande comportant une chambre de compression annulaire à volume réglable, reliée par un cathéter de réglage à un dispositif de réglage de la pression interne de ladite chambre, pour régler son expansion diamétrale.

### TECHNIQUE ANTERIEURE

Dans le cas de patients atteints d'obésité extrêmement sévère (obésité morbide), c'est-à-dire dans le cas de patients dont le poids excède le poids idéal d'au moins cinquante kilos par exemple, il est absolument nécessaire d'intervenir de manière chirurgicale sur de tels patients afin d'éviter non seulement une série de problèmes de santé découlant de cette obésité, mais encore pour éviter une mort certaine et proche de tels patients.

En effet, il est acquis que les patients souffrant d'obésité morbide voient leur espérance de vie réduite de manière importante et d'au moins une dizaine à une quinzaine d'années, tout en créant d'importants problèmes de charges psychologiques. Par ailleurs, toute une série de phénomènes annexes de santé sont impliqués, ayant une incidence sur l'apparition de maladies cardio-vasculaires, d'hypertension, de diabète, d'arthrites sévères notamment.

Il est également apparu que des traitements basés sur une diète sévère combinée avec une série d'exercices physiques associés à une modification du comportement, notamment alimentaire, étaient peu adaptés à de tels cas d'obésité morbide, bien que de telles méthodes de traitement soient les plus saines.

C'est la raison pour laquelle les traitements efficaces et à long terme de traitement de l'obésité morbide font intervenir un traitement chirurgical.

De manière générale, on distingue les techniques de traitement chirurgical faisant intervenir un défaut d'absorption des aliments, c'est-à-dire un raccourcissement du passage classique de l'aliment et des sucs digestifs et les techniques faisant intervenir une restriction gastrique, réduisant la taille de l'estomac.

Les techniques chirurgicales impliquant un défaut d'absorption sont par exemple celles impliquant une technique de by-pass ou dérivation du petit intestin ou encore celles mettant en oeuvre une séparation du passage des aliments relativement aux sucs digestifs. La technique chirurgicale de by-pass peut donner lieu à de sévères complications, de telle sorte que cette technique n'est plus que très rarement utilisée. La technique chirurgicale de séparation de passage du bol alimentaire relativement aux sucs digestifs ne fait pas intervenir de complications particulières, mais nécessite une ntervention chirurgicale importante impliquant notamment une gastrectomie partielle.

C'est la raison pour laquelle on tend désormais à utiliser les techniques chirurgicales mettant en oeuvre une restriction gastrique pour réduire la prise d'aliments.

De telles techniques font intervenir de manière classique l'utilisation d'anneaux de gastroplastie implantés autour de l'estomac pour réduire sa taille et le diamètre de son passage (stoma).

La plupart des dispositifs de gastroplastie connus, et par exemple celui décrit dans le brevet US-A-5074868 (dont l'objet correspond au préambule de la revendication 1 annexée ci-après), mettent en oeuvre une bande souple réalisée en matériau élastomère et destinée à être implantée autour de l'estomac puis resserrée et fermée suivant une boucle de diamètre fixe par un système de fermeture. Le corps de la bande souple comporte une cavité ou chambre de compression à volume variable reliée par un cathéter de réglage à un dispositif de réglage de la pression interne de la chambre afin de faire varier le diamètre interne de la boucle pour modifier ou régler le diamètre du stoma par injection ou extraction d'un volume de liquide hors de la chambre. Une telle opération de réglage du diamètre interne de l'anneau s'effectue à l'aide de dispositifs classiques de commande incluant un boîtier miniaturisé implanté directement sous la peau du patient et pourvu d'une membrane auto-obturante à travers laquelle le médecin injecte ou retire du liquide par une seringue.

Le système de fermeture du brevet US-A-5074868 met en oeuvre un suturage, à l'aide de fils de suture, des deux brins de la bande souple de l'anneau.

Un tel dispositif donne généralement satisfaction, mais souffre comme la plupart des systèmes connus, d'inconvénients liés essentiellement à la difficulté de toute intervention chirurgicale susceptible de survenir après la pose de l'implant de gastroplastie. En effet, il s'avère que malgré la possibilité de modifier dans une certaine mesure le diamètre de l'anneau sans intervention chirurgicale par le boîtier miniaturisé mentionné ci-dessus, la pose de tels implants gastriques peut s'accompagner de phénomènes d'intolérance, par exemple accompagné de vomissements, liés à une trop forte réduction du diamètre de stoma, ou encore à une action inefficace de l'implant lié à un diamètre du stoma trop important, ou encore tout simplement à une gêne ou une infection ou inflammation locale ou générale.

C'est la raison pour laquelle il est souvent nécessaire d'intervenir à nouveau de manière chirurgicale, soit pour soulager le patient, soit pour modifier ou changer l'anneau de gastroplastie préalablement implanté. De telles interventions chirurgicales sont particulièrement sévères et nécessitent de plus, soit la découpe de l'anneau par un chirurgien, soit comme dans le cas du brevet US-A-5074868 la coupe du fil de suture s'accompagnant d'une ouverture complète de l'anneau puis son changement et son remplacement.

En définitive, de telles opérations sont délicates à réaliser, difficilement supportées par le patient, coûteuses, et ce d'autant plus qu'elles impliquent la destruction d'un implant et son remplacement. En outre, dans le cas du brevet US-A-5074868, le dispositif d'aide à la coupe de la suture rend l'implant chirurgical relativement complexe à fabriquer et réaliser, sans pour autant constituer une aide essentielle lors de l'opération.

Le modèle d'utilité DE-G-9014048 présente un système partiellement annulaire spécifiquement conçu et adapté à la fermeture totale de vaisseaux sanguins, dont l'enseignement n'est en outre pas transposable à la compression de l'estomac qui doit être parfaitement maîtrisée.

### EXPOSE DE L'INVENTION

L'objet assigné à l'invention vise en conséquence à proposer un nouvel anneau de gastroplastie permettant de porter remède aux différents inconvénients énumérés précédemment et susceptible de faciliter l'intervention chirurgicale ainsi que d'éventuelles ré-interventions chirurgicales après la pose de l'implant, ne nécessitant pas le remplacement de l'implant, ce dernier étant dans tous les cas, de conception particulièrement simple et facile à réaliser.

Un autre objet de l'invention vise à proposer un nouvel anneau de gastroplastie susceptible d'assurer de manière simple et fiable une fermeture réversible de la boucle constituant l'anneau.

Un autre objet de l'invention vise à proposer un nouvel anneau de gastroplastie susceptible de proposer des moyens simples et fiables d'adaptation et de réglage affiné du diamètre de l'anneau à chaque situation chirurgicale donnée.

Un autre objet de l'invention vise à proposer un nouvel anneau de gastroplastie susceptible de présenter plusieurs diamètres d'implantation.

Un autre objet de l'invention vise à proposer un nouvel anneau de gastroplastie permettant de faciliter l'enfilage du cathéter.

Un autre objet de l'invention vise à proposer un nouvel anneau de gastroplastie particulièrement facile à fabriquer tout en étant d'une excellente résistance mécanique générale.

Les objets assignés à l'invention sont atteints à l'aide d'un anneau de gastroplastie formé par une bande souple destinée à être fermée autour de l'estomac vers ses deux parties d'extrémité par un système de fermeture pour réduire le diamètre de l'ouverture du stoma, ladite bande comportant une chambre de compression annulaire à volume réglable et à section sensiblement constante reliée par un cathéter de réglage à un dispositif de réglage de la pression interne de ladite chambre, pour régler son expansion diamétrale, caractérisé en ce que le système de fermeture comporte des moyens de blocage et desserrage de l'anneau qui sont portés par le cathéter de réglage, permettant d'assurer, à partir de leur position de blocage diamétral, le relâchement diamétral de l'anneau par déplacement relatif des deux parties d'extrémité.

### DESCRIPTIF SOMMAIRE DES DESSINS

D'autres objets et avantages de l'invention apparaîtront mieux à la lecture de la description ci-jointe de l'une de ses formes de réalisation, ainsi qu'à l'aide des dessins annexés, à titre purement illustratif et informatif dans lesquels :
- La figure 1 illustre selon une vue schématique en perspective, un exemple de réalisation d'un anneau de gastroplastie conforme à l'invention en position de fermeture.
- La figure 2 illustre selon une vue schématique en perspective, un exemple de réalisation d'un anneau de gastroplastie conforme à l'invention en position d'ouverture avant son implantation.
- La figure 3 illustre selon une vue en coupe transversale, la section d'un anneau de gastroplastie conforme à l'invention.

### MEILLEURE MANIERE DE REALISER L'INVENTION

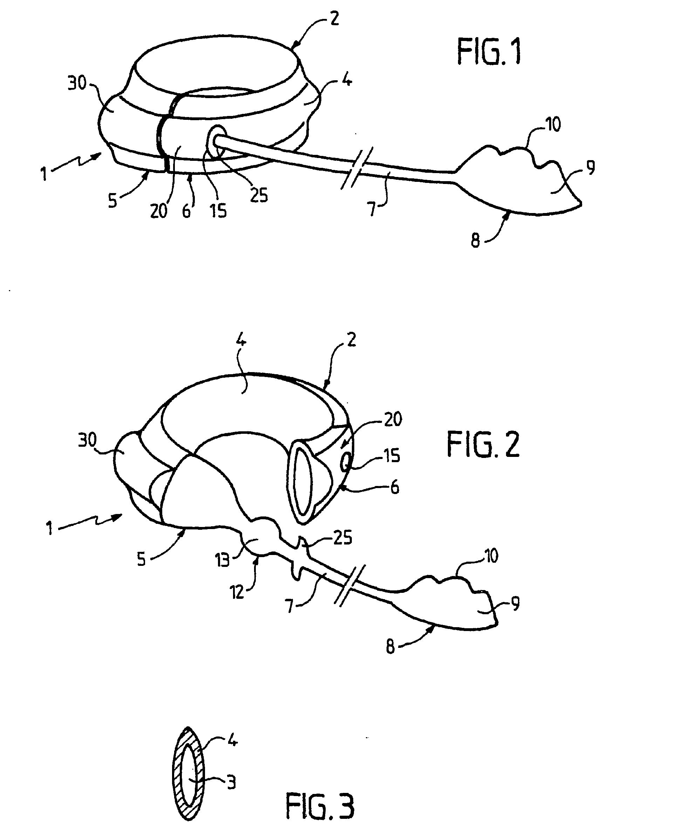

Les figures 1 et 2 illustrent un exemple de réalisation préférentielle d'un anneau de gastroplastie 1 conforme à l'invention, formé par une bande souple 2 réalisée par exemple par thermoformage à partir d'un matériau élastomère à usage chirurgical, ladite bande 2 définissant, de préférence sensiblement sur toute sa longueur, une chambre de compression interne 3, délimitée par les parois 4 de la bande souple 2 et par deux parties d'extrémité 5, 6. Dans sa position d'implantation dans l'estomac d'un patient, tel qu'illustré à la figure 1, la chambre de compression 3 forme donc une chambre de compression annulaire dont la section et le profil sont adaptés pour éviter la formation d'une zone de pincement susceptible de générer des plis avec les parois de l'estomac. Ceci implique notamment une surface de contact continue et uniforme avec l'estomac et la formation d'une zone de compression annulaire presque parfaite excluant notamment une forme en gouttelette, susceptible d'agresser les tissus cellulaires.

Tel que cela est bien connu dans l'art antérieur, la chambre de compression 3 définit un volume fermé et interne à l'anneau de gastroplastie, qui est destiné à former un volume réglable de manière à régler l'expansion diamétrale de l'anneau lorsqu'il est en place pour l'adapter à chaque situation chirurgicale donnée.

De manière classique, le réglage de l'expansion diamétrale de l'anneau de gastroplastie conforme à l'invention est assuré par un cathéter de réglage 7 formé par un élément tubulaire en matériau élastomère prolongeant l'une des parties d'extrémité libre, par exemple la partie d'extrémité 5, de la chambre de compression 3 pour relier ladite chambre à un dispositif de réglage 8 de la pression interne de ladite chambre.

Tel que cela est bien connu de l'homme de l'art, le dispositif de réglage 8 peut être formé par un boîtier miniaturisé 9 implanté sous la peau du patient. Le boîtier miniaturisé 9 comporte par exemple une membrane supérieure 10 auto-obturante destinée à être percée par une seringue pour injecter ou retirer une certaine quantité de fluide (eau distillée par exemple) servant à assurer la variation de volume de la chambre de compression 3, pour régler le volume de la chambre et obtenir ainsi le diamètre interne de l'anneau souhaité. Un tel dispositif étant bien connu de l'homme du métier, il ne sera en conséquence pas décrit plus en détail.

L'anneau de gastroplastie conforme à l'invention comporte également un système de fermeture destiné à fermer et maintenir en position de boucle l'anneau de gastroplastie autour de l'estomac.

Selon une caractéristique importante de l'invention, le système de fermeture conforme à l'invention comporte des moyens de blocage et desserrage de l'anneau qui sont portés par le cathéter de réglage 7, c'est à dire faisant partie intégrante de ce dernier et permettant d'assurer, à partir de la position de blocage diamétral de l'anneau correspondant à la position fermée et en boucle tel qu'illustré à la figure 1, son relâchement diamétral momentané par déplacement relatif des deux parties d'extrémité 5, 6 de l'anneau, tout en formant si on le souhaité, une boucle fermée autour de l'estomac. Avantageusement, les moyens de blocage et desserrage sont réversibles.

Selon l'invention, les moyens de blocage et de desserrage comportent des moyens pneumatiques pour assurer la fermeture et l'ouverture de l'anneau, faisant intervenir un fluide, un gaz ou un liquide par exemple. Le recours à des moyens pneumatiques permet une mise en place simplifiée de l'implant et une commande simple de son ouverture et fermeture.

La réalisation d'une telle fonction technique permet, en plus de la simplicité de mise en place et fermeture de l'anneau qu'elle procure, de réduire la gravité et l'importance d'éventuelles ré-interventions chirurgicales suivant la pose d'un implant en évitant d'avoir à sectionner et détruire l'anneau de gastroplastie mis en place. Une telle fonction permet de laisser l'anneau en place et d'assurer une simple augmentation momentanée de son diamètre sans détruire la boucle de l'anneau, ce qui permet un resserrage ultérieur pour revenir à la position de fermeture illustré à la figure 1.

Selon une variante préférentielle de l'invention, tel qu'illustré aux figures 1 et 2, l'anneau de gastroplastie conforme à l'invention comprend un système de fermeture dont les moyens de blocage et desserrage réversibles comportent au moins une zone déformable 12 formée ou réalisée sur le cathéter de réglage 7, et une ouverture 15 ménagée dans la paroi 4 de la partie d'extrémité 6 de la bande 2. Le cathéter de réglage 7 gonflable qui est réalisé en un matériau élastomère biocompatible, est destiné à être enfilé dans l'ouverture 15 dans la position de fermeture de l'anneau et à servir en outre de moyen de guidage.

La zone déformable 12 est susceptible de former une excroissance 13 en cas d'augmentation de pression dans le cathéter de réglage 7, ladite excroissance 13 prenant appui contre les parois 4 de la partie d'extrémité 6 de la bande, à l'intérieur de la chambre 3 pour bloquer l'anneau en position de fermeture. L'excroissance 13 reprend sa forme de repos en cas de retour à la pression normale dans le cathéter de réglage 7 pour permettre le coulissement et le guidage libre dudit cathéter dans l'ouverture 15 et le desserrage de la boucle.

Selon une version particulièrement avantageuse de l'invention, la zone déformable réversible 12 est formée par au moins une zone 13 de moindre résistance et dans sa forme de repos, constitue un relief par rapport au reste de la section du cathéter de réglage 7. En d'autres termes, la section transversale de la zone déformable 12 au repos, est d'une surface supérieure à celle de la section transversale du reste de la longueur du cathéter de réglage 7 de manière à former une sorte de légère boursouflure à la surface du cathéter.

Avantageusement, la zone déformable 12 peut être formée par une section du cathéter de réglage 7 présentant une dureté du matériau élastomère composant le cathéter, qui est localement inférieure à la dureté générale dudit cathéter. Dans un tel cas, le cathéter de réglage 7 étant relié au dispositif de réglage 8 externe de mise en pression par l'intermédiaire d'un fluide (air ou liquide), la zone 12 tendra à former un ballonnet 13 d'un diamètre supérieur aux dimensions intérieures de la bande, ce qui assure le blocage diamétral de l'anneau. La mise en pression du ballonnet 13 intervient avant celle de la bande.

Bien évidemment, à titre de variante, un anneau de gastroplastie conforme à l'invention pourra comprendre plusieurs zones 12 de moindre résistance, espacées de manière régulière ou non le long du cathéter de réglage 7 pour constituer un anneau de gastroplastie susceptible de comporter plusieurs diamètres fixes de pose.

Avantageusement, la bande souple 2 est pourvue à une portion d'extrémité, et par exemple à la portion d'extrémité 6 opposée à la portion d'extrémité 5 qui est prolongée par le cathéter de réglage 7, d'un manchon creux 20 prolongeant la bande souple 2.

Le manchon creux 20 comporte également l'ouverture 15 qui est ménagée dans l'une de ses faces, de préférence une face externe, de telle manière que l'autre extrémité 5 de la bande souple 2 puisse s'insérer dans ledit manchon en position de fermeture, le cathéter de réglage 7 passant alors dans l'ouverture 15 pour former la boucle de l'anneau. Une telle disposition constructive assure une bonne fiabilité de la tenue de la fermeture, tout en permettant à la chambre de compression 2 de s'étendre sur tout le périmètre de serrage de l'estomac.

De préférence, la bande 2 et le manchon creux 20 sont de section transversale de forme oblongue pour faciliter encore le coincement de la zone déformable 12 lors de sa mise en pression.

Avantageusement, la bande souple 2, la chambre de compression 3 ainsi que le manchon creux 20 forment une pièce monobloc réalisée à partir d'un même matériau plastique élastomère, le cathéter de réglage 7 étant ensuite thermosoudé.

De manière avantageuse, il est encore possible de réaliser la zone déformable 12 avec une épaisseur différente de l'épaisseur de la bande souple 2 afin d'obtenir des débits de fluide différents dans chacun de ces éléments. Ces débits différents peuvent également être obtenus par des formes différentes et ce, afin par exemple de dégonfler la bande souple 2 avant la zone déformable 12.

La partie d'extrémité 5 de la bande 2 à laquelle est relié le cathéter de réglage 7, est de préférence de forme conique pour faciliter son insertion dans le manchon creux 20. La zone déformable 12 est située au voisinage de la partie rétrécie de cette forme conique.

De préférence, le cathéter de réglage 7 possède des moyens de repérage visuel 25 qui sont situés à distance de la zone déformable 12 et qui sont destinés, en position de blocage de l'excroissance 13, à traverser l'ouverture 15 du manchon creux 20. Ces moyens de repérage visuel 25 consistent par exemple en une bague de diamètre supérieur à celui du cathéter. Ils permettent de vérifier que le cathéter a été suffisamment enfilé dans l'ouverture 15 et en outre à assurer un maintien en position de l'excroissance 13 bloquée dans le manchon creux 20.

En outre, le cathéter de réglage 7 est sensiblement rigide et est d'une longueur importante par rapport au diamètre de l'anneau pour faciliter l'opération d'enfilage et le passage du cathéter 7 dans l'ouverture 15. Ceci permet également une bonne mise en place de la bande dans la position désirée. Pour faciliter l'enfilage, il est possible de réaliser le cathéter de réglage 7, avec une portion d'extrémité de dureté augmentée relativement à sa portion adjacente à l'excroissance 13, la portion de dureté augmentée étant ensuite sectionnée après enfilage.

Pour faciliter encore une bonne mise en place de la bande, celle-ci possède sur sa paroi 4 des moyens de préhension 30 qui consistent en un surplus de matière placé à la surface de cette paroi.

Dans une variante de réalisation non représentée aux figures, le système de fermeture comporte en outre un moyen femelle placé à l'intérieur du manchon creux 20 en vue d'améliorer encore le blocage de l'excroissance 13. Selon cette variante, le moyen femelle prend la forme d'un oeillet contre lequel l'excroissance 13, ou le ballonnet, vient s'expanser et se bloquer en position, sous l'effet de la pression.

La réalisation d'un anneau de gastroplastie monobloc permet de simplifier le procédé de fabrication de l'anneau et d'obtenir un anneau ne présentant pas de risque d'altération dans le temps.

Lors de l'implantation, l'anneau conforme à l'invention est mis en place autour de l'estomac dans la positon illustrée à la figure 2. Le dispositif de réglage 8 étant au préalable déconnecté, le chirurgien assure l'enfilage et le passage du cathéter de réglage 7 dans l'ouverture 15 pour insérer l'extrémité 5 dans le manchon creux 20 (figure 1). Au besoin, le chirurgien réduit ensuite la longueur du cathéter de réglage 7 en sectionnant la portion de dureté augmentée repérée par exemple par un repère visuel. Le chirurgien peut ensuite, par l'intermédiaire du dispositif de réglage et de gonflage 8 raccordé à l'unique cathéter 7, assurer le blocage en position de l'anneau, en ayant au préalable veillé à positionner la zone de moindre résistance 12 correspondant au diamètre de l'implant souhaité, à l'intérieur du manchon 20, un contrôle visuel étant effectué au moyen de la bague 25. Le chirurgien peut ensuite assurer le réglage du diamètre interne de l'anneau en injectant ou retirant la quantité de liquide idoine au travers du cathéter 7. On notera que l'ensemble de ces opérations est réalisé au moyen d'un seul et unique cathéter 7.

En cas de ré-opération chirurgicale, il est possible grâce à l'anneau de gastroplastie conforme à l'invention, de se limiter à réaliser, par coelioscopie ou laparoscopie, un examen superficiel extérieur de la situation de l'implant, par simple inspection optique à l'aide d'une caméra. Eventuellement, si la situation le nécessite, il est possible dans un premier temps, par simple coelioscopie de mettre en dépression le cathéter 7, ce qui conduit à un relâchement du ballonnet 13 et à un coulissement du cathéter à travers l'ouverture 15 en raison de la longueur suffisante dudit cathéter. Un tel coulissement s'accompagne d'un desserrage partiel et momentané de l'anneau, sans avoir à opérer le patient de manière sévère. Il est ensuite possible, également par simple examen et intervention laparoscopique, de renfermer et bloquer l'anneau en position de fermeture de manière très simple, puisque la boucle de l'anneau n'a jamais été détruite.

### POSSIBILITE D'APPLICATION INDUSTRIELLE

L'invention trouve son application industrielle dans la réalisation et l'utilisation d'anneaux de gastroplastie.

## Revendications

1. Anneau de gastroplastie formé par une bande souple (2) destinée à être fermée autour de l'estomac vers ses deux parties d'extrémité (5, 6) par un système de fermeture (7, 12, 13, 15, 20) pour réduire le diamètre de l'ouverture du stoma, ladite bande comportant une chambre de compression (3) annulaire à volume réglable reliée par un cathéter de réglage (7) à un dispositif de réglage (8) de la pression interne de ladite chambre, pour régler son expansion diamétrale, **caractérisé en ce que** le système de fermeture (7, 12, 13, 15, 20) comporte au moins une zone déformable (12) réalisée sur le cathéter de réglage (7), ladite bande (2) étant pourvue d'un manchon creux (20) comportant une ouverture (15), le cathéter de réglage (7) étant apte à coulisser dans l'ouverture (15) pour former la boucle de l'anneau, la zone déformable (12) étant susceptible d'une part, en cas d'augmentation de pression dans le cathéter (7), de prendre appui contre les parois intérieures du manchon (20) pour bloquer l'anneau en position de fermeture, et d'autre part de reprendre sa forme de repos en cas de retour à la pression normale, pour permettre le coulissement libre du cathéter de réglage (7) et le desserrage de la boucle.

2. Anneau selon la revendication 1 **caractérisé en ce que** la chambre (3) présente une section sensiblement constante.

3. Anneau selon la revendication 1 ou 2, **caractérisé en ce que** la zone déformable (12) est susceptible d'une part de former localement une excroissance (13) en cas d'augmentation de pression dans le cathéter (7), et de prendre appui contre les parois intérieures de la bande (2) pour bloquer l'anneau en position de fermeture, et d'autre part de reprendre sa forme de repos en cas de retour à la pression normale, pour permettre le coulissement libre du cathéter de réglage (7) et le desserrage de la boucle.

4. Anneau selon l'une des revendications 1 à 3, **caractérisé en ce que** la zone déformable (12) est une zone de moindre résistance formée sur le cathéter de réglage (7).

5. Anneau selon l'une des revendications 1 à 4 **caractérisé en ce que** la zone déformable (12) est, dans sa forme de repos, en relief par rapport au reste de la section du cathéter de réglage (7).

6. Anneau selon l'une des revendications 1 à 5, **caractérisé en ce que** ladite première partie d'extrémité (6) de la bande souple (2) est pourvue dudit manchon creux (20).

7. Anneau selon l'une des revendications 1 à 6, **caractérisé en ce que** ladite deuxième partie d'extrémité (5) de la bande souple (2) est de forme convergente.

8. Anneau selon l'une des revendication 1 à 7, **caractérisé en ce que** le cathéter de réglage (7) comporte en outre des moyens de repérage visuel (25) qui sont situés à distance de la zone déformable (12) et qui sont destinés, lorsque la zone déformable (12) prend appui contre les parois intérieures du manchon (20) pour bloquer l'anneau en position de fermeture, à traverser l'ouverture (15) du manchon (20).

9. Anneau selon l'une des revendications 1 à 6, **caractérisé en ce que** les moyens de blocage et de desserrage (12) comportent des moyens pneumatiques faisant intervenir un fluide, un gaz ou un liquide.

10. Anneau selon l'une quelconque des revendications 1 à 9, **caractérisé en ce que** la section transversale de la bande (2) est de forme oblongue.

11. Anneau selon l'une quelconque des revendications 1 à 10 , **caractérisé en ce que** le cathéter de réglage (7) est sensiblement rigide et est d'une longueur importante par rapport au diamètre de l'anneau pour faciliter le passage du cathéter (7) dans l'ouverture (15).

12. Anneau selon l'une quelconque des revendications 1 à 11, **caractérisé en ce que** la bande (2) comporte, sur sa paroi extérieure, des moyens de préhension (30) destinés à faciliter la manipulation de l'anneau.

13. Anneau selon l'une quelconque des revendications 1 à 12, **caractérisé en ce que** le système de fermeture comporte en outre un moyen femelle solidaire de la bande (2) et monté sur ou relativement à cette dernière, les moyens de blocage et desserrage (12, 13) étant susceptibles de coulisser dans le moyen femelle entre une position de blocage et une position de desserrage.

14. Anneau selon l'une des revendications 1 à 13 , **caractérisé en ce que** le cathéter de réglage (7) est relié à un dispositif de mise en compression / décompression (8).

## Claims

1. Gastroplasty ring formed by a flexible band (2) which is designed to be closed around the stomach towards its two end parts (5, 6) by a closure system (7, 12, 13, 15, 20) so as to reduce the diameter of the opening of the stoma, said band comprising an annular compression chamber (3) of adjustable volume which is connected by an adjustment catheter (7) to a device (8) for adjusting the internal pressure of said chamber, so as to adjust its diametral expansion, **characterised in that** the closure system (7, 12, 13, 15, 20) comprises at least one deformable zone (12) formed on the adjustment catheter (7), said band (2) being provided with a hollow sleeve (20) comprising an opening (15), the adjustment catheter (7) being able to slide in the opening (15) so as to form the loop of the ring, the deformable zone (12) being able on the one hand, when the pressure in the catheter (7) is increased, to bear against the inner walls of the sleeve (20) so as to lock the ring in the closed position, and on the other hand to reassume its rest shape upon a return to normal pressure, so as to allow the free sliding of the adjustment catheter (7) and the release of the loop.

2. Ring according to claim 1, **characterised in that** the chamber (3) has a substantially constant cross section.

3. Ring according to claim 1 or 2, **characterised in that** the deformable zone (12) is able on the one hand to form locally a bulge (13) when the pressure in the catheter (7) is increased, and to bear against the inner walls of the band (2) so as to lock the ring in the closed position, and on the other hand to reassume its rest shape upon a return to normal pressure, so as to allow the free sliding of the adjustment catheter (7) and the release of the loop.

4. Ring according to one of claims 1 to 3, **characterised in that** the deformable zone (12) is a zone of reduced resistance which is formed on the adjustment catheter (7).

5. Ring according to one of claims 1 to 4, **characterised in that** the deformable zone (12) is, in its rest shape, in relief with respect to the rest of the cross section of the adjustment catheter (7).

6. Ring according to one of claims 1 to 5, **characterised in that** said first end part (6) of the flexible band (2) is provided with said hollow sleeve (20).

7. Ring according to one of claims 1 to 6, **characterised in that** said second end part (5) of the flexible band (2) is of convergent shape.

8. Ring according to one of claims 1 to 7, **characterised in that** the adjustment catheter (7) also comprises visual reference means (25) which are located at a distance from the deformable zone (12) and which are intended, when the deformable zone (12) bears against the inner walls of the sleeve (20) so as to lock the ring in the closed position, to pass through the opening (15) of the sleeve (20).

9. Ring according to one of claims 1 to 6, **characterised in that** the locking and release means (12) comprise pneumatic means which involve a fluid, a gas or a liquid.

10. Ring according to any one of claims 1 to 9, **characterised in that** the cross section of the band (2) is of oblong shape.

11. Ring according to any one of claims 1 to 10, **characterised in that** the adjustment catheter (7) is substantially rigid and is of considerable length compared to the diameter of the ring so as to facilitate the passage of the catheter (7) into the opening (15).

12. Ring according to any one of claims 1 to 11, **characterised in that** the band (2) comprises, on its outer wall, gripping means (30) which are designed to facilitate the handling of the ring.

13. Ring according to any one of claims 1 to 12, **characterised in that** the closure system also comprises a female means secured to the band (2) and installed on or relative to the latter, the locking and release means (12, 13) being able to slide in the female means between a locked position and a released position.

14. Ring according to one of claims 1 to 13, **characterised in that** the adjustment catheter (7) is connected to a compression/decompression device (8).

## Patentansprüche

1. Ring zur Magenverengung, gebildet aus einem elastischen Band (2), dafür vorgesehen, in Richtung auf seine zwei Endabschnitte (5, 6) durch ein Verschlusssystem (7, 12, 13, 15, 20) um den Magen geschlossen zu werden, um den Durchmesser der Öffnung des Stomas zu reduzieren, wobei das Band eine ringförmige Druckkammer (3) mit regulierbarem Volumen, verbunden durch cinen Regelkatheter (7) mit einer Vorrichtung zur Regelung (8) des inneren Drucks der Kammer, aufweist, um seine diametrale Ausdehnung zu regeln, **dadurch gekennzeichnet, dass** das Verschlusssystem (7, 12, 13, 15, 20) mindestens einen verformbaren Bereich (12) aufweist, der auf dem Regelkatheter (7) ausgebildet ist, wobei das Band (2) mit einer hohlen Muffe (20) versehen ist, die eine Öffnung (15) aufweist, wobei der Regelkatheter (7) dazu geeignet ist, in die Öffnung (15) zu gleiten, um die Ringschleife zu bilden, wobei der verformbare Bereich (12) dazu geeignet ist, sich einerseits, im Fall der Erhöhung des Drucks im Katheter (7) gegen die Innenwände der Muffe (20) anzulegen, um den Ring in der Verschlussposition zu blockieren, und andererseits für den Fall einer Rückkehr zum Normaldruck seine Ruheform wieder einzunehmen, um das freie Gleiten des Regelkatheters (7) und das Öffnen der Schleife zu ermöglichen.

2. Ring nach Anspruch 1, **dadurch gekennzeichnet, dass** die Kammer (3) einen im Wesentlichen konstanten Querschnitt aufweist.

3. Ring nach Anspruch 1 oder 2, **dadurch gekennzeichnet, dass** der verformbare Bereich (12) dazu geeignet ist, einerseits im Fall einer Erhöhung des Drucks im Katheter (7) lokal einen Auswuchs (13) zu bilden und sich gegen die Innenwände des Bandes (2) anzulegen, um den Ring in der Verschlussposition zu blockieren, und andererseits für den Fall einer Rückkehr zum Normaldruck seine Ruheform wieder einzunehmen, um das freie Gleiten des Regelkatheters (7) und das Öffnen der Schleife zu ermöglichen.

4. Ring nach einem der Ansprüche 1 bis 3, **dadurch gekennzeichnet, dass** der verformbare Bereich (12) ein Bereich geringeren Widerstandes, gebildet auf dem Regelkatheter (7), ist.

5. Ring nach einem der Ansprüche 1 bis 4, **dadurch gekennzeichnet, dass** der verformbare Bereich (12) in seiner Ruheform bezüglich des restlichen Abschnittes des Regelkatheters (7) erhaben ist.

6. Ring nach einem der Ansprüche 1 bis 5, **dadurch gekennzeichnet, dass** der erste Endabschnitt (6) des flexiblen Bandes (2) mit der hohlen Muffe (20) versehen ist.

7. Ring nach einem der Ansprüche 1 bis 6, **dadurch gekennzeichnet, dass** der zweite Endabschnitt (5) des flexiblen Bandes (2) eine konvergierende Form aufweist.

8. Ring nach einem der Ansprüche 1 bis 7, **dadurch gekennzeichnet, dass** der Regelkatheter (7) außerdem Mittel der visuellen Kennzeichnung (25) aufweist, die sich im einem Abstand vom verformbaren Bereich (12) befinden, und die, wenn sich der verformbare Bereich (12) gegen die Innenwände der Muffe (20) anlegt, um den Ring in der Verschlussstellung zu blockieren, dazu vorgesehen sind, die Öffnung (15) der Muffe (20) zu durchqueren.

9. Ring nach einem der Ansprüche 1 bis 6, **dadurch gekennzeichnet, dass** die Blockier- und Öffnungsmittel (12) pneumatische Mittel umfassen, die ein Fluid, ein Gas oder eine Flüssigkeit intervenieren lassen.

10. Ring nach einem der Ansprüche 1 bis 9, **dadurch gekennzeichnet, dass** der Querschnitt des Bandes (2) eine längliche Form aufweist.

11. Ring nach einem der Ansprüche 1 bis 10, **dadurch gekennzeichnet, dass** der Regelkatheter (7) im Wesentlichen starr ist und bezogen auf den Durchmesser des Ringes eine beträchtliche Länge aufweist, um den Durchgang des Katheters (7) durch die Öffnung (15) zu ermöglichen.

12. Ring nach einem der Ansprüche 1 bis 11, **dadurch gekennzeichnet, dass** das Band (2) auf seiner Außenwand Mittel zum Greifen (30) umfasst, die dazu vorgesehen sind, die Handhabung des Ringes zu erleichtern.

13. Ring nach einem der Ansprüche 1 bis 12, **dadurch gekennzeichnet, dass** das Verschlusssystem außerdem ein Hohlmittel umfasst, das mit dem Band (2) fest verbunden ist und auf oder im Verhältnis zu Letzterem montiert ist, wobei die Blockier- und öffnungsmittel (12, 13) dazu geeignet sind, in dem Hohlmittel zwischen einer Blockierposition und einer Öffnungsposition zu gleiten.

14. Ring nach einem der Ansprüche 1 bis 13, **dadurch gekennzeichnet, dass** der Regelkatheter (7) mit einer Vorrichtung zur Komprimierung / Dekomprimierung (8) verbunden ist.
